# EUROPEAN PATENT APPLICATION

(11) **EP 4 026 524 A1**
(43) Date of publication of application: **13.07.2022**
(21) Application number: 20861863.7
(22) Date of filing: 01.09.2020
(51) Int. Cl.: A61F 9/00, A61N 1/10

(54) **METHOD AND DEVICE FOR PREVENTION OR TREATMENT OF CATARACT**

(30) Priority: 05.09.2019 JP 2019162244
(71) Applicant: Sharp Kabushiki Kaisha, Sakai-shi Osaka 590-8522 (JP)
(72) Inventor: YAMAMOTO Satohiko, Sakai City Osaka 590-8522 (JP); TOMIMOTO Kimiaki, Sakai City Osaka 590-8522 (JP); KUBO Yukihiro, Sakai City Osaka 590-8522 (JP); FUNAMORI Hirokazu, Sakai City Osaka 590-8522 (JP); TANIGUCHI Taizou, Kobe City Hyogo 651-0085 (JP); KATAGIRI Minoru, Kobe City Hyogo 651-0093 (JP); MATSUMOTO Kinuyo, Kobe City Hyogo 650-0046 (JP); TAKENOKUCHI Mariko, Kobe City Hyogo 650-0023 (JP)
(74) Representative: Treeby, Philip David William
(86) International application number: PCT/JP2020/032972
(87) International publication number: WO 2021/045023

(57) **Abstract**

A medical device according to the present invention is a medical device that includes an ion generating unit configured to generate a positive ion and a negative ion by discharge, and that prevents or treats a cataract by delivering the positive ion and the negative ion to a surface of an eye.

## Description

### Technical Field

One aspect of the present invention relates to a method for preventing or treating a cataract and a medical device for preventing or treating the cataract. The present application claims priority from JP 2019-162244 filed in Japan on September 5, 2019, the content of which is hereby incorporated by reference into this application.

### Background Art

A cataract is a disease in which a crystalline lens of an eye becomes opaque causing visual impairment. The cataract has been known to appear due to accumulation of stress (for example, ultraviolet stress, oxidation stress, glycation stress, and the like) on an eyeball. A number of research for a purpose of preventing or treating the cataract has been performed.

For example, PTL 1 describes cataract prevention food containing a collagen peptide. PTL 2 describes pharmaceutical preparation for preventing a cataract containing thalidomide as an active ingredient. PTL 3 describes an oral cataract prevention agent containing an yeast extract as an active ingredient.

### Citation List

### Patent Literature

PTL 1: JP 2012-100612 A (published on May 31, 2012)
PTL 2: JP 2006-169183 A (published on June 29, 2006)
PTL 3: WO 2017/209336 (published on December 7, 2017)

### Summary of Invention

### Technical Problem

As described above, development of food or pharmaceutical preparation for preventing a cataract is proceeding. On the other hand, development of a method for preventing or treating a cataract by direct action on an eye, such as contact with an eye including an affected area of a cataract, is not greatly proceeding. Further, development of a device for preventing or treating a cataract is also not proceeding.

Therefore, an object according to one aspect of the present invention is to achieve a new method and a new device for preventing or treating a cataract.

### Solution to Problem

In order to solve the problem described above, a method according to one aspect of the present invention is a method including preventing or treating a cataract of a non-human mammal by delivering a positive ion and a negative ion to a surface of an eye of the non-human mammal.

Further, a medical device according to one aspect of the present invention is a medical device that includes an ion generating unit configured to generate a positive ion and a negative ion by discharge, and that prevents or treats a cataract by delivering the positive ion and the negative ion to a surface of an eye.

### Advantageous Effects of Invention

One aspect of the present invention can prevent or treat a cataract.

### Brief Description of Drawings

FIG. 1 is a diagram illustrating an overall configuration of a medical device according to a first embodiment of the present invention.
FIG. 2 is a diagram illustrating an overall configuration of a medical device according to a second embodiment of the present invention.
FIG. 3 is a diagram illustrating an overall configuration of a medical device according to a third embodiment of the present invention.
FIG. 4 is a diagram illustrating an overall configuration of a medical device according to a fourth embodiment of the present invention.
FIG. 5 is a diagram illustrating an overall configuration of a medical device according to a fifth embodiment of the present invention.
FIG. 6 is a diagram illustrating an overall configuration of a medical device according to a sixth embodiment of the present invention.
FIG. 7 is a diagram illustrating an overall configuration of a medical device according to a seventh embodiment of the present invention.
FIG. 8 is a diagram illustrating an overall configuration of a medical device according to an eighth embodiment of the present invention.
FIG. 9 is a diagram illustrating an overall configuration of a medical device according to a ninth embodiment of the present invention.
FIG. 10 is a diagram illustrating an overall configuration of a medical device according to a tenth embodiment of the present invention.
FIG. 11 is a diagram illustrating an overall configuration of a medical device according to an eleventh embodiment of the present invention.
FIG. 12 is a diagram illustrating an overall configuration of a medical device according to a twelfth embodiment of the present invention.
FIG. 13 is a picture illustrating the front of an experimental device used in an example.
FIG. 14 is a picture illustrating the top of the experimental device used in the example.

### Description of Embodiments

### First Embodiment

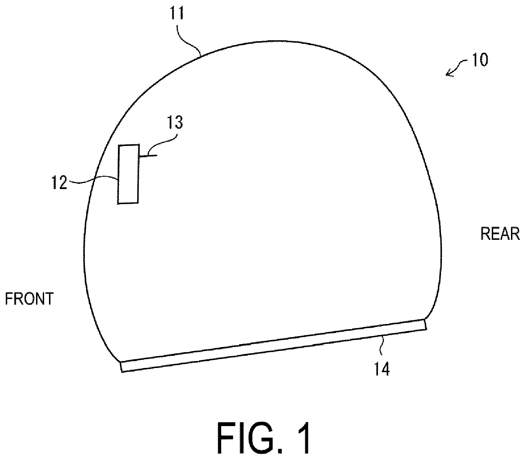

Hereinafter, a first embodiment according to the present invention will be described in detail based on FIG. 1. FIG. 1 is a diagram illustrating an overall configuration of a medical device 10 according to the first embodiment.

A cataract is a disease in which a crystalline lens of an eye becomes opaque causing visual impairment. The cataract is considered to appear due to stress (for example, ultraviolet stress, oxidation stress, glycation stress, and the like) on an eyeball. Examples of the ultraviolet stress include irradiation with ultraviolet light or radiation, and the like. Examples of the oxidation stress include smoking, and the like. Examples of the glycation stress include a lifestyle-related disease such as diabetes, and the like.

### Medical Device 10

As illustrated in FIG. 1, the medical device 10 includes a cover 11 that covers an eye of a subject, and an ion generator 12 (ion generating unit) provided inside the cover 11. Examples of the subject include mammals such as humans.

### Cover 11

The cover 11 is a member for forming a space that covers the eye of the subject. An opening portion 14 for inserting a head including the eye of the subject into the cover 11 is provided in the cover 11. The head of the subject is inserted into the cover 11 from the opening portion 14, and thus the eye of the subject can be covered with an internal space of the cover 11.

Then, a high concentration of positive ions and negative ions generated from the ion generator 12 described below fill the internal space of the cover 11, and the positive ions and the negative ions are delivered to a surface of the eye. In this way, a high concentration of the positive ions and the negative ions can directly act on the eye. Then, the cataract can be prevented, or an affected area of the cataract can be treated.

A shape of the cover 11 in FIG. 1 is a substantially hemispherical shape, but the shape is not particularly limited as long as the eye of the subject can be covered with the internal space of the cover 11. A size of the cover 11 is also not particularly limited as long as the eye of the subject can be covered with the internal space of the cover 11. For example, the cover 11 may cover a part of the head of the subject, or may cover the entire head of the subject. Further, a material of the cover 11 is not particularly limited as long as the material does not have an influence when the positive ion and the negative ion generated from the ion generator 12 described below fill the internal space of the cover 11. Examples of the material of the cover 11 include, for example, a resin and the like.

### Ion Generator 12

The ion generator 12 is a member that generates the positive ion and the negative ion. The ion generator 12 includes a positive ion generating unit (not illustrated), a negative ion generating unit (not illustrated), and a voltage supply unit (not illustrated) that supplies a voltage to the positive ion generating unit and the negative ion generating unit. Each of the positive ion generating unit and the negative ion generating unit includes an induction electrode (not illustrated) and a discharge electrode 13 having a needle shape. The discharge electrode 13 is provided so as to protrude from the ion generator 12 toward the internal space of the cover 11. Note that, for convenience of illustration, FIG. 1 illustrates only the discharge electrode 13 of one of the ion generating units.

In the positive ion generating unit, by applying a positive voltage from the voltage supply unit, a water molecule in the atmosphere (air) is electrically decomposed in a plasma region generated by discharge. Then, a hydrogen ion H⁺ is mainly generated. Then, a positive cluster ion H⁺(H₂O)ₘ (where m is any integer) is formed by aggregation of the water molecule in the air around the generated hydrogen ion H⁺.

In the negative ion generating unit, by applying a negative voltage from the voltage supply unit, an oxygen molecule in the atmosphere (the air) is electrically decomposed in a plasma region generated by discharge. Then, an oxygen ion O₂⁻ is mainly generated. Then, a negative cluster ion O₂⁻(H₂O)ₙ (where n is any integer) is formed by aggregation of the water molecule in the air around the generated oxygen ion O₂⁻.

Further, an active species OH radical (·OH) and the like are generated by a reaction of the positive cluster ion H⁺(H₂O)ₘ (where m is any integer) and the negative cluster ion O₂⁻(H₂O)ₙ (where n is any integer).

A configuration of the ion generator 12 described above is merely one example, and is not particularly limited as long as the ion generator 12 is a device that can generate the positive ion and the negative ion at a desired concentration by discharge.

In order for a high concentration of the positive ions and the negative ions to easily act directly on the eye of the subject in a position located away from the plasma region generated by discharge, the ion generator 12 is provided inside the cover 11 so as to be disposed near the eye.

In a viewpoint of causing a high concentration of the positive ions and the negative ions to directly act on the eye of the subject, and the like, each concentration of the positive ions and the negative ions generated by discharge is preferably equal to or greater than 500000/cm³, and is more preferably equal to or greater than one million/cm³. The ion concentration refers to an ion concentration delivered from the ion generator 12 or an ion concentration in the internal space of the cover 11. The ion concentration is acquired by counting a small ion as a target, and critical mobility in the air is set to be 1 cm²/V·s.

Whether to include the positive ion and the negative ion in a specific space can be determined by inspecting a gas composition by a gas mass analysis inspection, a gas concentration inspection, a discoloration inspection, an odor inspection, a light emission inspection, a generated sound inspection, and the like. A known mass analysis device can be used for the gas mass analysis inspection, and gas chromatography and an ion counter can be used for a measurement in the gas concentration inspection. Further, the discoloration inspection and the odor inspection can be performed in a sensory inspection such as visual determination and an olfactory inspection, and can also use a color difference meter, an odor sensor, and the like. Further, the light emission inspection and the generated sound inspection can be performed in a sensory inspection such as visual determination and an auditory inspection, and can also use an absorptiometer, a spectrograph, an optical sensor, an illuminance meter, a microphone, and the like.

### Second Embodiment

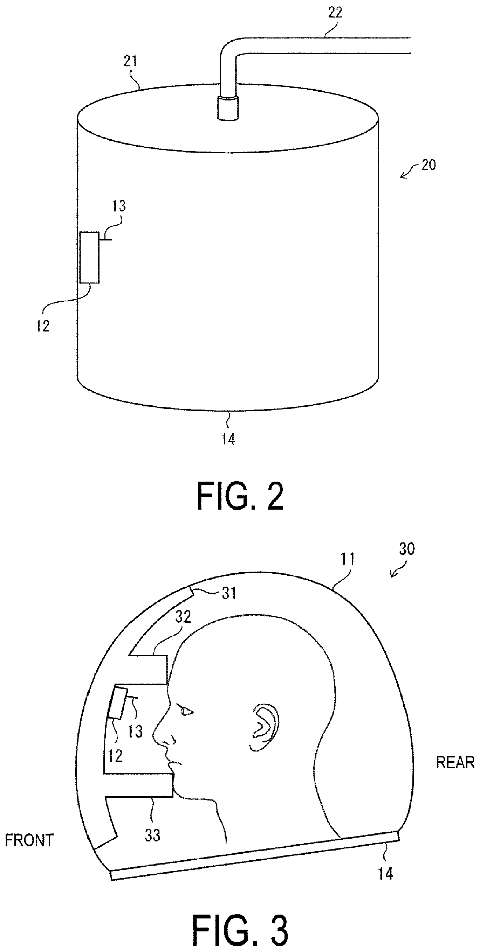

Hereinafter, a second embodiment according to the present invention will be described in detail based on FIG. 2. FIG. 2 is a diagram illustrating an overall configuration of a medical device 20 according to the second embodiment. Note that, for convenience of description, components having the same function as that of components described in the above embodiment are designated by the same reference numerals, and the descriptions of these components will be omitted.

As illustrated in FIG. 2, a cover 21 of the medical device 20 has a substantially cylindrical shape. Further, a fixing portion 22 is provided on an upper portion of the cover 21.

One end of the fixing portion 22 may be fixed to the upper portion of the cover 21, and another end may be fixed to a wall, a ceiling, and the like. By fixing the fixing portion 22 to the wall, the ceiling, and the like, a subject does not need to support the cover 21, and a load on the subject can be reduced.

### Third Embodiment

Hereinafter, a third embodiment according to the present invention will be described in detail based on FIG. 3. FIG. 3 is a diagram illustrating an overall configuration of a medical device 30 according to the third embodiment. Note that, for convenience of description, components having the same function as that of components described in the above embodiment are designated by the same reference numerals, and the descriptions of these components will be omitted. The present embodiment relates to a configuration in which a protection portion 31 is added to the medical device 10 according to the first embodiment.

### Protection Portion 31

The protection portion 31 is a member provided inside a cover 11 and used for preventing a discharge electrode 13 of an ion generator 12 from coming into contact with a forehead of a subject. The protection portion 31 may be provided on a part of an inner surface of the cover 11, or may be provided on the entire inner surface. Examples of a material of the protection portion 31 include a resin and the like.

A forehead placement portion 32 and a jaw placement portion 33 are provided on the protection portion 31. The forehead placement portion 32 and the jaw placement portion 33 are members for preventing the discharge electrode 13 of the ion generator 12 from coming into contact with the subject. Further, the ion generator 12 is provided between the forehead placement portion 32 and the jaw placement portion 33.

The forehead placement portion 32 and the jaw placement portion 33 are provided so as to protrude from the protection portion 3 1 toward an internal space of the cover 11. With the configuration, when the subject wears the medical device 30, a forehead and a jaw of the subject come into contact with the forehead placement portion 32 and the jaw placement portion 33, respectively, and thus a risk that the subject comes into contact with the discharge electrode 13 having the needle shape of the ion generator 12 can be reduced.

### Fourth Embodiment

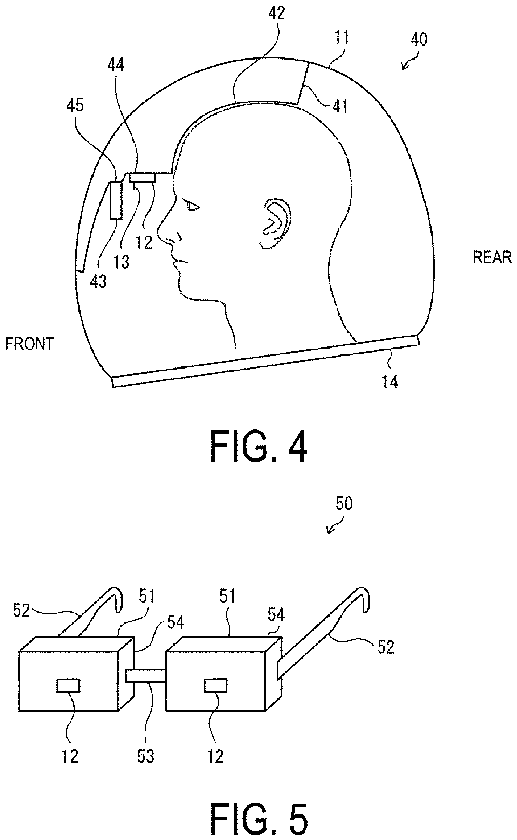

Hereinafter, a fourth embodiment according to the present invention will be described in detail based on FIG. 4. FIG. 4 is a diagram illustrating an overall configuration of a medical device 40 according to the fourth embodiment. Note that, for convenience of description, components having the same function as that of components described in the above embodiment are designated by the same reference numerals, and the descriptions of these components will be omitted. The present embodiment relates to a configuration in which a protection portion 41 and an air blower 43 (an air sending unit) are added to the medical device 10 according to the first embodiment.

### Protection Portion 41

The protection portion 41 is a member provided inside a cover 11 and used for preventing a discharge electrode 13 of an ion generator 12 from coming into contact with a forehead of a subject. The protection portion 41 may be provided on a part of an inner surface of the cover 11, or may be provided on the entire inner surface. Examples of a material of the protection portion 41 include a resin and the like.

A head placement portion 42, an ion generator placement portion 44, and an air blower placement portion 45 are provided on the protection portion 41. The head placement portion 42 comes into contact with a head such as the forehead of the subject when the subject wears the medical device 40. The head placement portion 42 illustrated in FIG. 4 has a curved surface so as to easily fix the head of the subject inside the cover 11. The ion generator 12 is installed on the ion generator placement portion 44. In the configuration illustrated in FIG. 4, the ion generator placement portion 44 is provided near the head placement portion 42 such that the ion generator 12 is disposed near an eye of the subject. The ion generator placement portion 44 is provided closer to an inner surface side of the cover 11 than the head placement portion 42 in order to reduce a risk that the subject comes into contact with the discharge electrode 13 having the needle shape of the ion generator 12. The air blower 43 described below is installed on the air blower placement portion 45. In order to deliver, from the ion generator 12 to a surface of the eye of the subject, a positive ion and a negative ion generated from the ion generator 12, the air blower placement portion 45 is provided near the ion generator placement portion 44. Further, the air blower placement portion 45 is provided closer to the inner surface side of the cover 11 than the ion generator placement portion 44.

### Air Blower 43

The air blower 43 is a member for delivering an ion generated from the ion generator 12 to the surface of the eye of the subject. The air blower 43 generates, inside the cover 11, an air flow for delivering the positive ion and the negative ion generated from the ion generator 12 from the ion generator 12 to the surface of the eye of the subject. The generation of the air flow causes a high concentration of the positive ions and the negative ions to easily act directly on the eye. The air blower 43 is not particularly limited as long as the air blower 43 is a device that generates the air flow for delivering, to the surface of the eye of the subject, the positive ion and the negative ion generated from the ion generator 12. Further, the air blower 43 may simultaneously deliver the positive ion and the negative ion, or may alternately deliver the positive ion and the negative ion.

In the medical device 40 in FIG. 4, the air blower 43 is provided on the protection portion 41 such that an air blowing surface is substantially perpendicular to a surface of the ion generator 12 on which the discharge electrode 13 is provided. Further, the air blower 43 is provided on the protection portion 41 such that an array direction of two discharge electrodes 13 of the ion generator 12 and an air blowing direction are substantially perpendicular to each other. The air blowing direction of the air blower 43 is substantially perpendicular to the array direction of the discharge electrodes 13 of the ion generator 12, and thus an ion loss caused by canceling out the positive ion and the negative ion generated from the ion generator 12 with each other is less likely to occur.

### Fifth Embodiment

Hereinafter, a fifth embodiment according to the present invention will be described in detail based on FIG. 5. FIG. 5 is a diagram illustrating an overall configuration of a medical device 50 according to the fifth embodiment. Note that, for convenience of description, components having the same function as that of components described in the above embodiment are designated by the same reference numerals, and the descriptions of these components will be omitted.

As illustrated in FIG. 5, the medical device 50 includes two covers 51, two temples 52, and a bridge 53. The bridge 53 is provided between the two covers 51 so as to couple the two covers 51. Each of the two covers 51 includes the temple 52 on a side surface opposite to a side surface coupled to the bridge 53. A space is provided inside the cover 51, and an ion generator 12 is provided inside the cover 51.

The medical device 50 in FIG. 5 is configured to cover a left eye and a right eye with the two corresponding covers 51, but when a cataract of one eye is prevented or treated, the cover 51 of the medical device 50 may be one. When the number of the cover 51 of the medical device 50 is one, a band instead of the temples 52 may be provided on both side surfaces of the cover 51 in order to fix the cover 51 to cover the eye.

A shape of the cover 51 in FIG. 5 is a substantially rectangular parallelepiped shape, but the shape is not particularly limited as long as the eye of the subject can be covered with an internal space of the cover 51.

An opening portion 54 for covering the eye of the subject with the internal space of the cover 51 is provided in the cover 51 on a side in contact with a face of the subject. The opening portion 54 has an opening area that can cover one eye of the subject. A sealing member may be provided on the opening portion 54 such that the cover 51 easily adheres to the face of the subject. Examples of a material of the sealing member include rubber, a resin, and the like.

### Sixth Embodiment

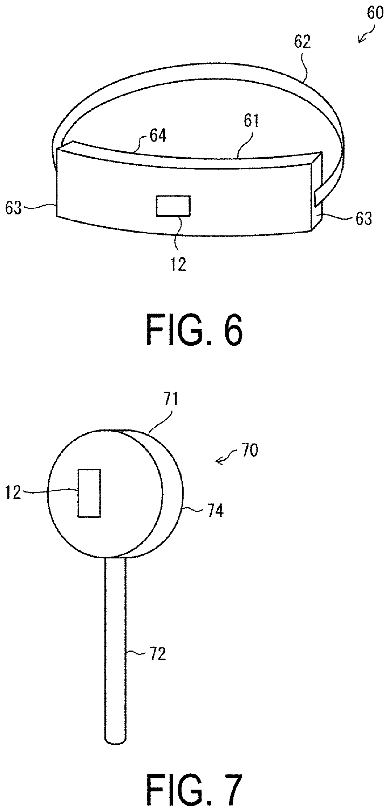

Hereinafter, a sixth embodiment according to the present invention will be described in detail based on FIG. 6. FIG. 6 is a diagram illustrating an overall configuration of a medical device 60 according to the sixth embodiment. Note that, for convenience of description, components having the same function as that of components described in the above embodiment are designated by the same reference numerals, and the descriptions of these components will be omitted.

As illustrated in FIG. 6, the medical device 60 includes a cover 61, an ion generator 12 provided inside the cover 61, and a band 62 provided on a side surface 63 of the cover 61.

An opening portion 64 for covering an eye of a subject with an internal space of the cover 61 is provided in the cover 61 on a side in contact with a face of the subject. The opening portion 64 has an opening area that can cover both eyes of the subject. In a viewpoint of causing a positive ion and a negative ion to uniformly act on both eyes of the subject, the ion generator 12 is provided at the center inside the cover 61.

The band 62 is a member for fixing the cover 61 to cover the eyes. The band 62 may include an adjustment member so as to be able to adjust a length according to a size of a head of the subject.

### Seventh Embodiment

Hereinafter, a seventh embodiment according to the present invention will be described in detail based on FIG. 7. FIG. 7 is a diagram illustrating an overall configuration of a medical device 70 according to the seventh embodiment. Note that, for convenience of description, components having the same function as that of components described in the above embodiment are designated by the same reference numerals, and the descriptions of these components will be omitted.

As illustrated in FIG. 7, the medical device 70 includes a cover 71, an ion generator 12 provided inside the cover 71, and a grip portion 72 provided on a side surface of the cover 71.

An opening portion 74 for covering an eye of a subject in an internal space of the cover 71 is provided in the cover 71 on a side in contact with a face of the subject. The opening portion 74 has an opening area that can cover the eye of the subject.

The grip portion 72 is a member for the subject to hold the medical device 70 with hand to cover the eye of the subject with the cover 71. The subject holds the grip portion 72 with hand and brings the opening portion 74 of the cover 71 into contact with the face of the subject, and thus the eye of the subject is covered with the internal space of the cover 71. The grip portion 72 preferably has a shape easily held by the subject with hand, and examples of the shape include a stick shape and the like.

### Eighth Embodiment

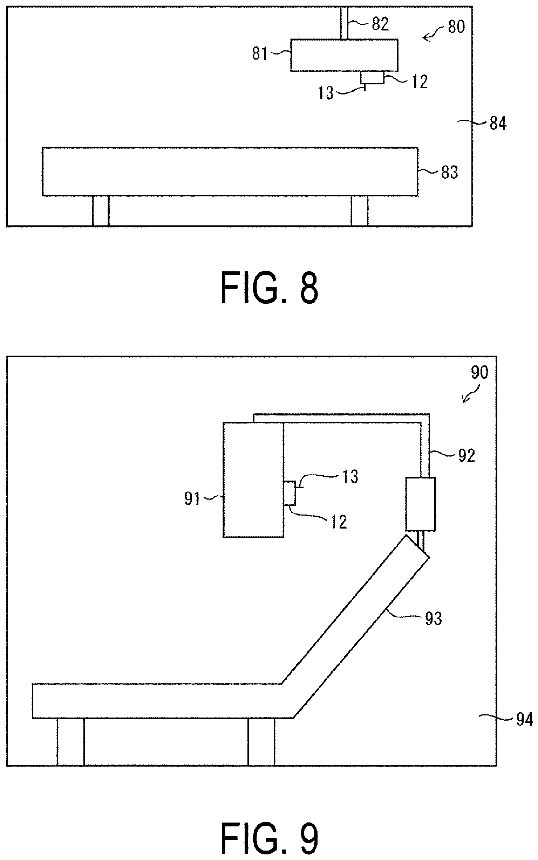

Hereinafter, an eighth embodiment according to the present invention will be described in detail based on FIG. 8. FIG. 8 is a diagram illustrating an overall configuration of a medical device 80 according to the eighth embodiment. Note that, for convenience of description, components having the same function as that of components described in the above embodiment are designated by the same reference numerals, and the descriptions of these components will be omitted.

As illustrated in FIG. 8, the medical device 80 includes an ion generator 12, an ion generator fixing portion 81 to which the ion generator 12 is fixed, and a hanging portion 82 that hangs the ion generator fixing portion 81 from a ceiling. A discharge electrode 13 protrudes, toward a floor, from a side surface opposite to a side surface of the ion generator 12 fixed to the ion generator fixing portion 81.

The medical device 80 is provided in a space 84. Examples of the space 84 include a living room and a bedroom in a standard home, a consultation room in a hospital, a conference room, and the like. In a viewpoint of causing a high concentration of positive ions and negative ions generated from the ion generator 12 to directly act on an eye of a subject, the space 84 is preferably a substantially closed space during use of the medical device 80.

A bed 83 is installed inside the space 84 in a vertically downward direction of the medical device 80. The subject lies face up on the bed 83 such that the ion generator 12 is located above the eye of the subject. Then, a high concentration of the positive ions and the negative ions generated from the ion generator 12 is delivered to a surface of the eye of the subject. In this way, a high concentration of the positive ions and the negative ions can directly act on the eye. Then, the cataract can be prevented, or an affected area of the cataract can be treated.

### Ninth Embodiment

Hereinafter, a ninth embodiment according to the present invention will be described in detail based on FIG. 9. FIG. 9 is a diagram illustrating an overall configuration of a medical device 90 according to the ninth embodiment. Note that, for convenience of description, components having the same function as that of components described in the above embodiment are designated by the same reference numerals, and the descriptions of these components will be omitted.

As illustrated in FIG. 9, the medical device 90 includes an ion generator 12, a bed 93, an ion generator fixing portion 91 to which the ion generator 12 is fixed, and a fixing portion 92 that fixes the ion generator fixing portion 91 to the bed 93. A discharge electrode 13 protrudes from a side surface opposite to a side surface of the ion generator 12 fixed to the ion generator fixing portion 91. Further, the medical device 90 is provided in a space 94. An example of the inside of the space 94 is the same as an example of that of the space 84.

In the medical device 90 in FIG. 9, the bed 93 is a reclining bed. An angle of the bed 93 is adjusted, and the ion generator 12 is provided in front of an eye of a subject.

### Tenth Embodiment

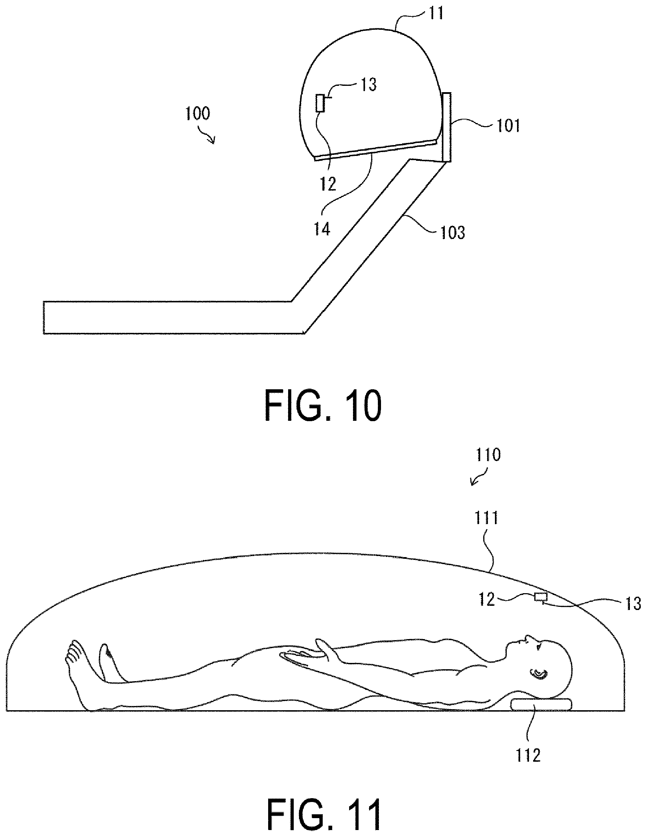

Hereinafter, a tenth embodiment according to the present invention will be described in detail based on FIG. 10. FIG. 10 is a diagram illustrating an overall configuration of a medical device 100 according to the tenth embodiment. Note that, for convenience of description, components having the same function as that of components described in the above embodiment are designated by the same reference numerals, and the descriptions of these components will be omitted. The present embodiment relates to a configuration in which a cover fixing portion 101 and a bed 103 are added to the medical device 10 according to the first embodiment.

As illustrated in FIG. 10, the medical device 100 further includes the cover fixing portion 101 and the bed 103. The bed 103 is a reclining bed. The cover fixing portion 101 is provided on an upper portion of the bed 103. In FIG. 10, an outer surface of a cover 11 is fixed to the cover fixing portion 101, but the cover 11 may be fixed to the bed 103 by fixing an inner surface of the cover 11 to the cover fixing portion 101.

A subject lies on the bed 103, and a head including an eye of the subject is inserted from an opening portion 14, and thus the eye is covered with an internal space of the cover 11.

### Eleventh Embodiment

Hereinafter, an eleventh embodiment according to the present invention will be described in detail based on FIG. 11. FIG. 11 is a diagram illustrating an overall configuration of a medical device 110 according to the eleventh embodiment. Note that, for convenience of description, components having the same function as that of components described in the above embodiment are designated by the same reference numerals, and the descriptions of these components will be omitted.

As illustrated in FIG. 11, the medical device 110 includes a cover 111 that covers a whole body of a subject, an ion generator 12 provided inside the cover 111, and a head protection member 112 that protects a head of the subject.

The cover 111 is a member for forming a space that covers an eye of the subject by covering the whole body of the subject. An entrance for the subject to enter the cover 111 may be provided in the cover 111. The ion generator 12 is installed above the eye of the subject (a face of the subject) such that the subject can lie inside the cover 111 to prevent or treat a cataract.

The head protection member 112 is disposed near a place directly below the ion generator 12 such that a positive ion and a negative ion generated from the ion generator 12 easily act directly on the eye of the subject. Examples of the head protection member 112 include a pillow and the like.

### Twelfth Embodiment

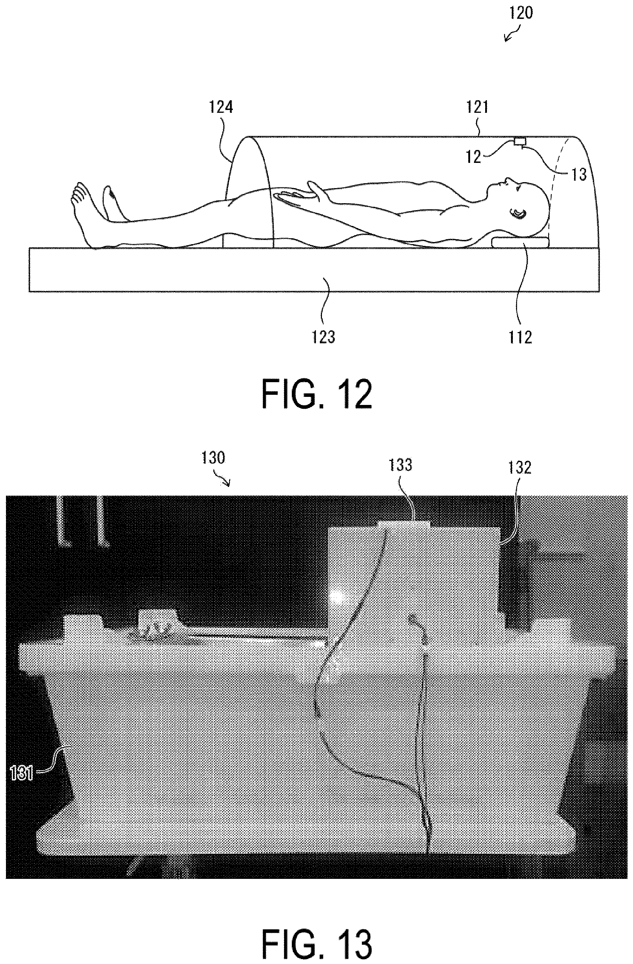

Hereinafter, a twelfth embodiment according to the present invention will be described in detail based on FIG. 12. FIG. 12 is a diagram illustrating an overall configuration of a medical device 120 according to the twelfth embodiment. Note that, for convenience of description, components having the same function as that of components described in the above embodiment are designated by the same reference numerals, and the descriptions of these components will be omitted.

As illustrated in FIG. 12, the medical device 120 includes a cover 121 that covers a subject, an ion generator 12 provided inside the cover 121, and a head protection member 112 that protects a head of the subject.

The cover 121 is a member for forming a space that covers an eye of the subject by covering a part of a body or a whole body of the subject. An opening portion 124 is provided on one side of the cover 121. The cover 121 is installed on the bed 123, and thus a space is provided inside the cover 121. The subject enters from the opening portion 124, and thus the eye of the subject can be covered with an internal space of the cover 121.

### Method for Preventing or Treating Cataract of Non-human Mammal

In a method for preventing or treating a cataract of a non-human mammal according to the present embodiment (hereinafter may be simply abbreviated as the "method according to the present embodiment"), a positive ion and a negative ion are delivered to a surface of an eye of the non-human mammal. In delivering the positive ion and the negative ion, the positive ion and the negative ion may be simultaneously delivered, or the positive ion and the negative ion may be alternately delivered. Each concentration of the positive ion and the negative ion to be delivered is preferably equal to or greater than 500000/cm³, and is more preferably equal to or greater than one million/cm³.

The positive ion and the negative ion can be generated by discharge, for example. The discharge may be performed in the atmosphere. Examples of the positive ion described above include a positive ion formed of H⁺(H₂O)ₘ (where m is any integer). Examples of the above negative ion described above include a negative ion formed of O₂⁻(H₂O)ₙ (where n is any integer).

Further, in the method according to the present embodiment, an eye of the non-human mammal may be covered to fill a covered space with the positive ion and the negative ion. At this time, each concentration of the positive ion and the negative ion in the covered space is preferably set to be equal to or greater than 500000/cm³, and is more preferably set to be equal to or greater than one million/cm³.

Furthermore, in the method for preventing or treating a cataract of a non-human mammal according to the present embodiment, the positive ion and the negative ion generated by discharge may be delivered to a surface of the eye of the non-human mammal in a position located away from a plasma region generated by the discharge. In this way, a high concentration of the positive ions and the negative ions can directly act on the eye of the non-human mammal.

In the method according to the present embodiment, a cataract of the non-human mammal can be prevented or treated by using the medical device described above.

Examples of the non-human mammal being a target of treatment or prevention in the method according to the present embodiment include cows, wild boars, pigs, sheep, goats, horses, mice, rats, hamsters, squirrels, rabbits, dogs, cats, ferrets, and the like. The non-human mammal may be a domestic animal or a pet animal, or may be a wild animal.

### Supplement

A method according to an aspect 1 of the present invention is a method for preventing or treating a cataract of a non-human mammal by delivering a positive ion and a negative ion to a surface of an eye of the non-human mammal.

According to the configuration described above, the positive ion and the negative ion can directly act on the eye of the non-human mammal, and the cataract can be prevented or treated.

In a method according to an aspect 2 of the present invention in the aspect 1, the eye of the non-human mammal may be covered to fill a covered space with the positive ion and the negative ion.

According to the configuration described above, the effect described in the aspect 1 can be acquired.

In a method according to an aspect 3 of the present invention in the aspect 1 or 2, each concentration of the positive ion and the negative ion may be equal to or greater than 500000/cm³.

According to the configuration described above, the effect described in the aspect 1 can be acquired.

The medical device 10 according to an aspect 4 of the present invention is a medical device that includes an ion generating unit (the ion generator 12) configured to generate a positive ion and a negative ion by discharge, and that prevents or treats a cataract by delivering the positive ion and the negative ion to a surface of an eye.

According to the configuration described above, the effect described in the aspect 1 can be acquired.

The medical device 10 according to an aspect 5 of the present invention in the aspect 4 may further include the cover 11 configured to cover the eye, and may deliver the positive ion and the negative ion to a covered space.

According to the configuration described above, the effect described in the aspect 1 can be acquired.

In the medical device 10 according to an aspect 6 of the present invention in the aspect 4 or 5, each concentration of the positive ion and the negative ion may be equal to or greater than 500000/cm³.

According to the configuration described above, the effect described in the first aspect described above can be acquired.

The present invention is not limited to each of the above-described embodiments. It is possible to make various modifications within the scope of the claims. An embodiment obtained by appropriately combining technical elements each disclosed in different embodiments falls also within the technical scope of the present invention. Furthermore, technical elements disclosed in the respective embodiments may be combined to provide a new technical feature.

### Examples

### Verification of Suppression of Occurrence of Cataract

When diabetes appears, it has been known that glycation stress is accumulated on an eyeball and an occurrence risk of a cataract increases. Thus, whether occurrence of a cataract is suppressed by causing a positive ion and a negative ion to act on a rat in which diabetes appears was verified.

### Experimental Animal

Type I diabetes was caused to appear in a rat by streptozotocin administration. Further, a mouse in which diabetes did not appear was used as a comparison.

### Experimental Device

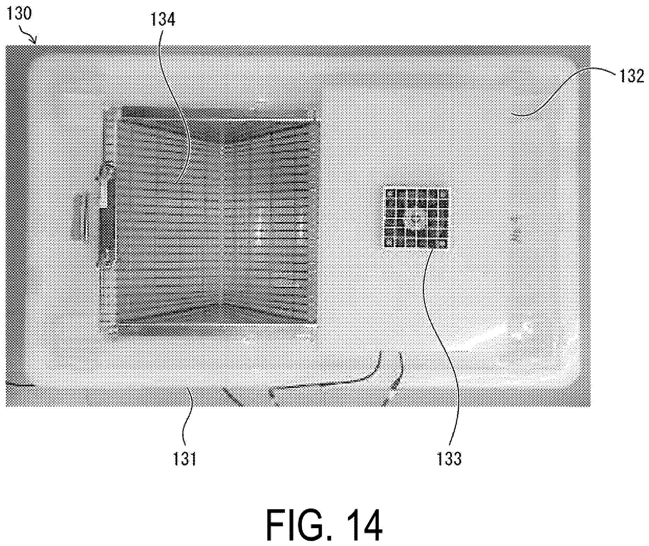

A medical device 130 illustrated in FIGS. 13 and 14 was used as an experimental device. FIG. 13 is a picture illustrating the front of the experimental device, and FIG. 14 is a picture illustrating the top of the experimental device. The experimental device (the medical device 130) includes a cage 131 made of plastic (305 mm long x 520 mm wide x 170 mm tall), and an ion generator 132 attached to a lid 134 of the cage 131. CKITTA252AKKZ manufactured by Sharp Co., Ltd. was used as the ion generator 132. Further, an air blower (a fan) 133 was provided on an upper portion of the ion generator 132 such that the inside of an entire cage 131 was filled with a positive ion and a negative ion that are generated. One rat was raised per one experimental device.

### Raising Condition

Rats in which diabetes appeared were divided into two groups. One group (diabetes/PCI (+) group) was raised for six weeks under an environment in which the positive ion and the negative ion were generated from the ion generator 132. During raising, a concentration of the positive ion and a concentration of the negative ion inside the cage 131 were adjusted to be one million/cm³. The ion concentration was measured in an appropriate range with a suction opening of an ion counter facing an upper portion of the cage 131.

Another group (diabetes/PCI (-) group) and a controlled group (normal/PCI (-) group) were raised inside the experimental device for six weeks without the positive ion and the negative ion being generated from the ion generator 132.

In each of the diabetes/PCI (+) group and the diabetes/PCI (-) group, verification with three rats was performed. In the normal/ PCI (-) group, verification with two rats was performed.

For each of the groups, CRF-1 (Oriental Yeast) was given as feed, and tap water was given as drinking water. Replacement of a floor mat in the cage 131 and water supply replenishment were performed once in four days. Further, a weight measurement, a symptom observation of a cataract, and cleaning of a discharge needle (discharge electrode) of the ion generator 132 were performed once a week. The symptom observation of the cataract was performed by observing a degree of opacity of an eyeball.

### Result of Cataract Symptom Observation

A result of the symptom observation of the cataract in each of the groups is shown in Table 1. In Table 1, a symptom confirmation date is illustrated by the number of elapsed days since an experiment start date.

**[Table 1]**

| GROUP | NO. | SYMPTOM CONFIRMATION DATE | SYMPTOM | |
|---|---|---|---|---|
| | | | LEFT EYE | RIGHT EYE |
| DIABETES/PCI (+) | 1 | - | - | - |
| | 2 | 51 | MILD | - |
| | 3 | - | - | - |
| DIABETES/PCI (-) | 1 | 49 | MILD | MILD |
| | 2 | 49 | MODERATE | - |
| | 3 | 39 | MODERATE (BECOMING SERIOUS LATER) | - |
| NORMAL/PCI (-) | 1 | - | - | - |
| | 2 | - | - | - |

Ozone is generated when the positive ion and the negative ion are generated. It has been initially expected that occurrence of the cataract would have been advanced due to the oxidation stress by the generated ozone. However, occurrence of the cataract was unexpectedly suppressed in the diabetes/PCI (+) group as compared to the diabetes/PCI (-) group in which the positive ion and the negative ion were not generated. Also in histological consideration performed by removing an eyeball from the rat in a seventh week since the experiment start date, occurrence of the cataract was suppressed in the diabetes/PCI (+) group as compared to the diabetes/PCI (-) group.

It was found from the result described above that occurrence of the cataract could be suppressed in a space in which the positive ion and the negative ion were generated.

### Industrial Applicability

The present invention can be used in, for example, treatment, research, and the like of a cataract.

## Claims

1. A method comprising:
preventing or treating a cataract of a non-human mammal by delivering a positive ion and a negative ion to a surface of an eye of the non-human mammal.

2. The method according to claim 1,
wherein the eye of the non-human mammal is covered and a covered space is filled with the positive ion and the negative ion.

3. The method according to claim 1 or 2,
wherein each concentration of the positive ion and the negative ion is equal to or greater than 500000/cm³.

4. A medical device comprising:
an ion generating unit configured to generate a positive ion and a negative ion by discharge,
wherein the medical device prevents or treats a cataract by delivering the positive ion and the negative ion to a surface of an eye.

5. The medical device according to claim 4, further comprising
a cover configured to cover the eye,
wherein the medical device delivers the positive ion and the negative ion to a covered space.

6. The medical device according to claim 4 or 5,
wherein each concentration of the positive ion and the negative ion is equal to or greater than 500000/cm³.
